# EUROPEAN PATENT SPECIFICATION

(11) **EP 4 469 459 B1**
(45) Date of publication and mention of the grant of the patent: **21.01.2026**
(21) Application number: 23702085.4
(22) Date of filing: 24.01.2023
(51) Int. Cl.: C07D 487/04

(54) **PROCESS FOR PREPARING CRYSTALLINE SITAGLIPTIN HYDROCHLORIDE MONOHYDRATE**
VERFAHREN ZUR HERSTELLUNG VON KRISTALLINEM SITAGLIZINHYDROCHLORID-MONOHYDRAT
PROCÉDÉ DE PRÉPARATION DE MONOHYDRATE D'HYDROCHLORURE DE SITAGLIPTINE CRISTALLIN

(30) Priority: 24.01.2022 EP 22460007
(43) Date of publication of application: 04.12.2024
(73) Proprietor: Zaklady Farmaceutyczne Polpharma S.A., 83-200 Starogard Gdanski (PL)
(72) Inventor: GRAUZENIS, Maciej, 83-200 Starogard Gdanski (PL); SAGOL, Karol, 83-110 Tczew (PL); HALUSZCZUK, Adam, 80-058 Gdansk (PL)
(74) Representative: Kondrat, Mariusz
(86) International application number: PCT/EP2023/051644
(87) International publication number: WO 2023/139276

(56) References cited:
- WO-A1-03/004498
- WO-A1-2005/072530
- WO-A1-2020/109938
- WO-A2-2012/025944
- CN-A- 110 857 302
- LIU FENG ET AL: "The asymmetric synthesis of Sitagliptin, a selective dipeptidyl peptidase IV inhibitor for the treatment of type 2 diabetes", JOURNAL OF CHEMICAL RESEARCH - SYNOPSES, SCIENCE REVIEWS LTD, GB, vol. 34, 1 April 2010 (2010-04-01), pages 230 - 232, XP009151355, ISSN: 0308-2342, DOI: 10.3184/030823410X12709912414009

## Description

### Technical Field

The present invention relates to the field of processes for preparing crystalline sitagliptin salts and hydrates thereof.

### Background art

Type 2 diabetes is a disease in which the pancreas does not make enough insulin to control the level of glucose in the blood or when the body is unable to use insulin effectively.

Sitagliptin is the INN of the compound (*R*)-4-oxo-4-[3-(trifluoromethyl)-5,6-dihydro[1,2,4]triazolo[4,3-a]pyrazin-7(8*H*)-yl]-1-(2,4,5-trifluorophenyl)butan-2-ami-ne of formula (I):

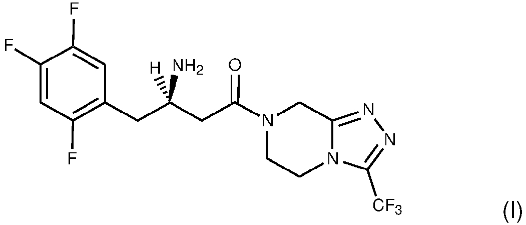

Sitagliptin was developed by the company Merck & Co and it was first disclosed in the international patent application WO-A-03/004498, published in 2003.

This active ingredient is a selective inhibitor of dipeptidyl peptidase IV (DPP-IV), which is used in medicine as antidiabetic. As disclosed in the EPAR summary for the public corresponding to the medicament Januvia^{®}, sitagliptin works by blocking the breakdown of 'incretin' hormones in the body. These hormones are released after a meal and stimulate the pancreas to produce insulin. By increasing the levels of incretin hormones in the blood, sitagliptin stimulates the pancreas to produce more insulin when blood glucose levels are high. Sitagliptin does not work when the blood glucose is low. Sitagliptin also reduces the amount of glucose made by the liver, by increasing insulin levels and decreasing the levels of the hormone glucagon.

Being sitagliptin a basic compound, pharmaceutically acceptable salts thereof sitagliptin may be prepared from pharmaceutically acceptable non-toxic acids, including inorganic and organic acids, such as, for example, acetic, benzenesulfonic, benzoic, camphorsulfonic, citric, ethanesulfonic, fumaric, gluconic, glutamic, hydrobromic, hydrochloric, isethionic, lactic, maleic, malic, mandelic, methanesulfonic, mucic, nitric, pamoic, pantothenic, phosphoric, succinic, sulfuric, tartaric, p-toluenesulfonic acid, and the like, as disclosed in WO'498.

In international patent applications WO-A-2012/025944 and WO-A-2015/170340 are disclosed different crystalline forms of sitagliptin hydrochloride.

In international patent application WO-A-2005/072530 it is disclosed the preparation of sitagliptin hydrochloride monohydrate from sitagliptin free base dissolved in a mixture of isopropanol and methanol. A solution of hydrochloric acid in diethyl ether was added and the solid in form of crystals was isolated, after heating to 55°C a thick slurry of crystals and slowly cooling down to room temperature. This process includes the use of diethyl ether, which is highly volatile, highly flammable and difficult to handle at industrial level.

In international patent application WO-A-2008/000418 it is disclosed the preparation of amorphous sitagliptin hydrochloride by adding by adding at least one equivalent of methanol and trimethylchlorosilane to a solution of sitagliptin free base in a mixture of dichloromethane and diethyl ether.

In international patent application WO-A-2010/000469 it is disclosed the preparation of sitagliptin hydrochloride forms I and II, wherein form I showed an increase in water content when stored at high humidity conditions (93 % RH, 2 weeks). Form I is prepared starting from sitagliptin free base in isopropanol solution by reaction with an aqueous solution of hydrochloric acid and recrystallized from ethanol. Form II is prepared by heating Form I in ethyl acetate, and subsequent cooling.

In international patent application WO-A-2010/012781 it is disclosed the preparation of an amorphous form of sitagliptin hydrochloride from a suspension of sitagliptin base in isopropanol, to which an aqueous solution of hydrochloric acid was added.

In international patent application WO-A-2011/123641 it is disclosed the preparation of amorphous sitagliptin hydrochloride from the free base dissolved in methanol, to which an aqueous solution of hydrochloric acid was added. It is further disclosed the preparation of sitagliptin hydrochloride forms IV and V, and crystalline sitagliptin hydrochloride monohydrate form III. The former compounds are obtained from amorphous sitagliptin hydrochloride stored in a glass vial under ethanol or isopropanol respectively, and the latter from sitagliptin free base dissolved in isopropanol and hydrochloric acid.

In Indian patent application 590/MUM/2011 it is disclosed a process for preparing crystalline hydrochloride monohydrate, which also includes a crystallization step with diethyl ether. Said process comprises dissolving sitagliptin free base in isopropanol/methanol followed by the addition of hydrochloric acid in isopropanol solution. After heating at 40-45°C, the product was obtained as crystals by adding diethyl ether.

In international patent application WO-A-2012/147092 it is disclosed a process for preparing sitagliptin hydrochloride starting from sitagliptin free base dissolved in methanol, heated up to 40-45°C, and treated with hydrochloric acid in isopropanol. After stirring at 25-30°C, heating up to 55°C, and cooling to 25-30°C, a solid was filtered.

In international patent application WO-A-2016/162877 it is disclosed the preparation of sitagliptin hydrochloride monohydrate starting from sitagliptin free base using different combinations of solvents. In an embodiment, the free base is dissolved in dichloromethane and water, and hydrochloric acid is added. In another embodiment, the free base is dissolved in ethyl acetate, hydrochloric acid in isopropanol is added, and water is added. In a further embodiment, the free base is dissolved in methyl ethyl ketone and water, hydrochloric acid in isopropanol is added. In another embodiment, the free base is dissolved in a mixture of ethyl acetate and ethanol, hydrochloric acid in isopropanol is added, and water is added. In all cases, crystalline sitagliptin hydrochloride monohydrate is obtained.

In international patent application WO-A-2020/109938 it is also disclosed the preparation of sitagliptin hydrochloride monohydrate starting from sitagliptin free base using different combinations of solvents. In one embodiment, sitagliptin free base is dissolved in a mixture of dichloromethane and isopropanol, hydrochloric acid in isopropanol is added, and methyl *tert*-butyl ether is added. In another embodiment, sitagliptin free base is dissolved in isopropanol, and hydrochloric acid in isopropanol is added. In another embodiment, water is added to a dichloromethane layer containing sitagliptin free base, hydrochloric acid in isopropanol is added, and isopropanol is further added. In another embodiment, sitagliptin free base is dissolved in dichloromethane, concentrated hydrochloric acid is added, the resultant mixture is distilled, a mixture of isopropanol and water is added, and methyl *tert*-butyl ether is added. In another embodiment, hydrochloric acid in isopropanol is added to a toluene layer containing Boc-sitagliptin (*tert*-butyl (*R*)-(4-oxo-4-(3-(trifluoromethyl)-5,6-dihydro-[1,2,4]triazolo[4,3-a]pyrazin-7(8*H*)-yl)-1-(2,4,5-trifluorophenyl)butan-2-yl)carbamate)), after stirring a complete distillation, isopropanol is added, and the mixture is seeded with sitagliptin hydrochloride monohydrate. In all cases, crystalline sitagliptin hydrochloride monohydrate was obtained.

Despite the various proposals available in the state of the art, there is still a need to have new processes for preparing sitagliptin hydrochloride monohydrate in an easy way being suitable for industrial production.

### Object of the invention

The object of the present invention is a process for preparing crystalline sitagliptin hydrochloride monohydrate.

### Figures

Figure 1

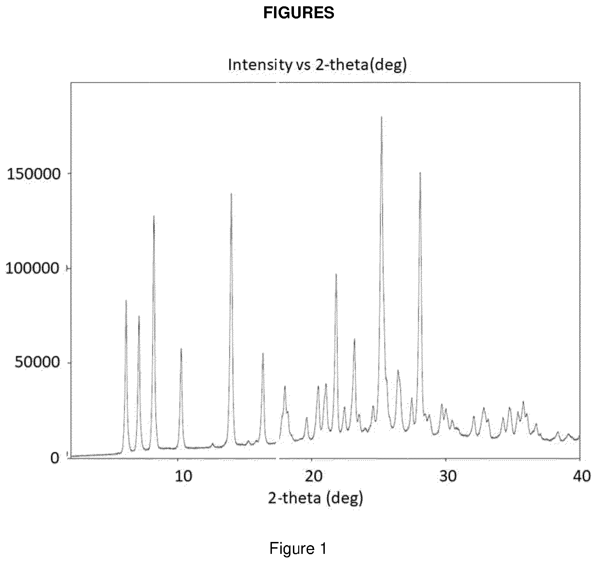

   Figure 1 shows the X-ray diffraction pattern of the crystalline ethanol solvate of sitagliptin hydrochloride obtained in step a) of the process of the invention.
Figure 2

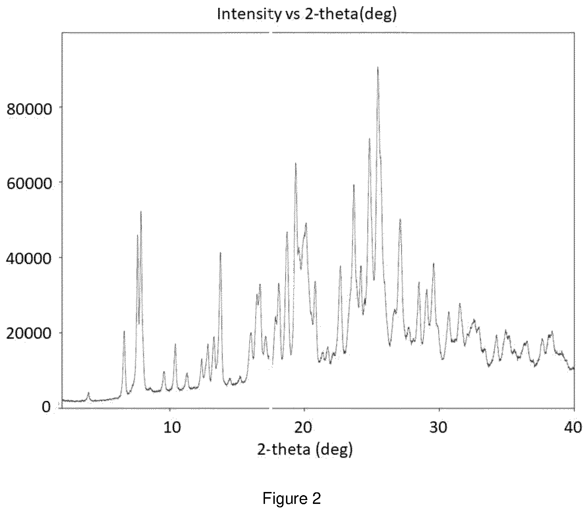

   Figure 2 shows the X-ray diffraction pattern of crystalline anhydrous sitagliptin hydrochloride, compound of formula (III), obtained in step b) of the process of the invention.
Figure 3

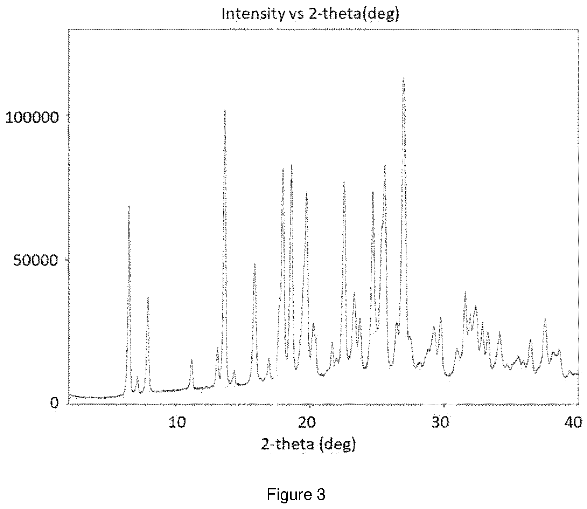

   Figure 3 shows the X-ray diffraction pattern of crystalline sitagliptin hydrochloride monohydrate, compound of formula (IV), obtained in step c) of the process of the invention.
Figure 4

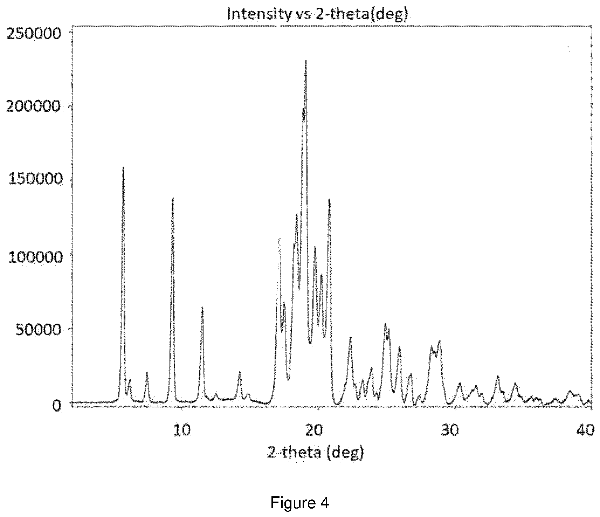

   Figure 4 shows the X-ray diffraction pattern of crystalline Boc-sitagliptin, compound of formula (II), obtained according to the process of the invention.

### Detailed description of the invention

The object of the present invention is a process for preparing crystalline sitagliptin hydrochloride monohydrate, which comprises:
a) deprotecting compound of formula (II) 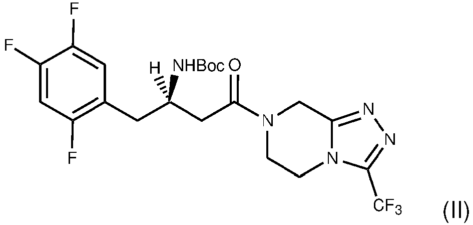 with a solution of hydrochloric acid or hydrogen chloride in a solvent to isolate a crystalline solid product;
b) drying the crystalline solid product obtained in step a) to obtain crystalline anhydrous sitagliptin hydrochloride of formula (III): 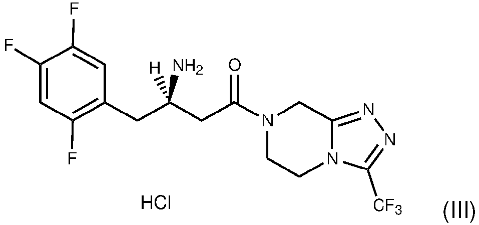
c) hydrating the product obtained in step b) to obtain crystalline sitagliptin hydrochloride monohydrate of formula (IV)

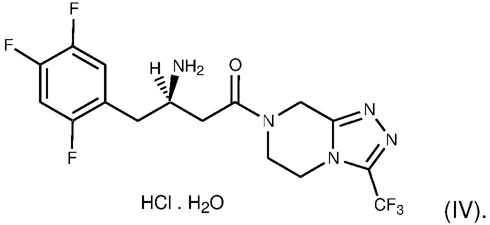

In an embodiment, after step c), the crystalline product may be conditioned in containers. The conditioning time may be set up by the skilled person in the art according to routine experiments. For example, the conditioning step may last at least 24h, for at least 36 h or for at least 48 h.

The authors of the present invention have developed a new process for preparing crystalline sitagliptin hydrochloride monohydrate starting from Boc-sitagliptin (compound of formula (II)), avoiding the isolation of sitagliptin free base, which is suitable for the industrial manufacturing of said product in high yield and purity. Said process provides sitagliptin hydrochloride monohydrate in a purity ranging between 99.9% and 100.0%, showing a significantly low content of impurities, wherein ethanol, isopropanol and water are used as solvents, instead of many toxic alternatives, and environmentally green coupling reagents are used, instead of many toxic alternatives.

In the present description, as well as in the claims, the singular forms "a", "an" and "the" include the plural reference unless the context clearly indicates otherwise. The ranges defined by the preposition the terms "between ... and ..." or by the terms "from ...to..." include also the two ends thereof. The term "about" refers to a deviation of plus/minus 10 %, preferably plus/minus 5 %. The percentages are expressed in % by weight, unless stated the contrary.

The process of the invention for preparing crystalline sitagliptin hydrochloride monohydrate starting from compound of formula (II) is represented in Scheme I:

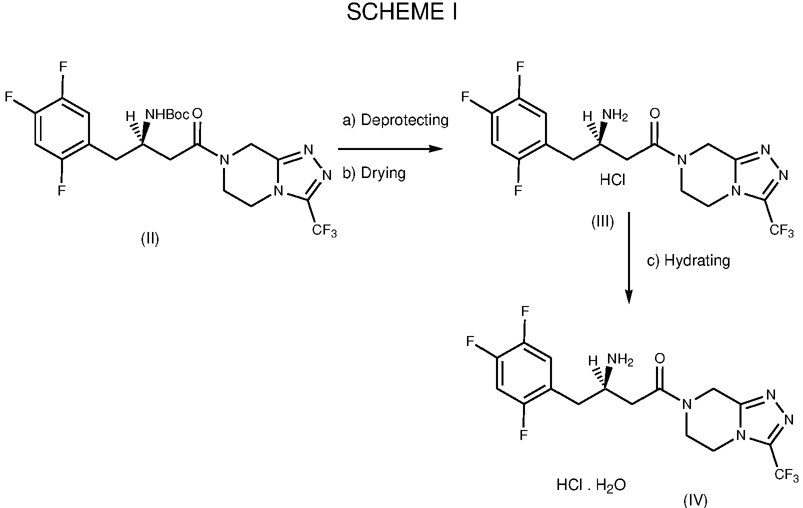

### Deprotection step

The Boc group (*tert*-butyloxycarbonyl) is used extensively in organic synthesis for amine protection. It is not hydrolysed under basic conditions and is inert to many other nucleophilic reagents.

The deprotection step of compound of formula (II) can be carried out under using different reagents under acid conditions, such as, for example, 3 M hydrochloric acid in ethyl acetate, acetyl chloride in methanol, trifluoroacetic acid in thiophenol, *p*-toluenesulfonic acid in a mixture of THF and dicloromethane, 100% sulfuric acid in dioxane, 0.05 M methanesulfonic acid in a mixture of dioxane and dichloromethane.

In the process of the invention, the deprotection of compound of formula (II) is carried out with a solution of hydrochloric acid or hydrogen chloride in a solvent. In an embodiment, the solvent is an organic solvent. The organic solvent may be selected from methanol, ethanol, isopropanol, dichloromethane, ethyl acetate, tetrahydrofuran, dioxane, thiophenol, or mixtures thereof. In a preferred embodiment the organic solvent is ethanol.

In an embodiment, the deprotection of compound of formula (II) is carried out by dissolving compound of formula (II) in ethanol and adding concentrated hydrochloric acid. In an embodiment, the acid concentration is comprised between 5% and 37%, preferably between 10% and 35%, more preferably between 31% and 35%, and yet more preferably about 33%.

In an embodiment, the deprotection of compound of formula (II) is carried out by dissolving compound of formula (II) in ethanol and adding a solution of hydrogen chloride in ethanol. In an embodiment, the concentration of hydrogen chloride is comprised between 5% and 37%, preferably between 10% and 35%, more preferably between 31% and 35%, and yet more preferably about 33%.

Deprotection can be carried out by heating the reaction mixture to a temperature comprised between 20°C and 90°C, preferably between 30°C and 85°C, preferably between 40°C and 80°C, and more preferably between 50°C and 75°C, and maintaining to said temperature for a period of time comprised between 2 h and 8 h, preferably between 4h and 7h, and more preferably about 6 h. Monitoring of deprotection can be carried out in an easy way by using TLC.

In an embodiment, the isolation of the solid product resulting from the deprotection step may be carried out using standard work-up procedures. For example, said isolation can include heating and/or cooling steps. In an embodiment, the isolation process may include adjusting the pH of the reaction mixture, for example, to a value comprised between 2.5 and 4.0.

In an embodiment, the isolation of the solid product resulting from the deprotection step comprises seeding the reaction mixture with crystals of sitagliptin hydrochloride monohydrate. In an embodiment, the isolation process comprises further a cooling step.

In an embodiment, the isolation of the solid product resulting from the deprotection step may comprise a combination of at least one heating step, a seeding, at least one cooling step, and filtering of the solid.

The conditions of the heating and cooling steps may be set up by the skilled person in the art using common general knowledge.

The solid product obtained by deprotection compound of formula (II) according to the process of the invention is a crystalline solid comprising an organic solvate of sitagliptin hydrochloride and optionally sitagliptin hydrochloride monohydrate. In an embodiment, said solid product is a crystalline solid comprising sitagliptin hydrochloride ethanol solvate and optionally sitagliptin hydrochloride monohydrate. The crystalline solid usually comprises at least 5 wt.%, at least 10 wt.%, at least 15 wt.%, at least 20 wt.%, at least 25 wt.%, at least 30 wt.%, at least 35 wt.%, at least 40 wt.%, at least 45 wt%., at least 50 wt.%, at least 55 wt.%, at least 60 wt.%, at least 65 wt.%, at least 70 wt.%, at least 75 wt.%, at least 80 wt.%, at least 85 wt.%, at least 90 wt.%, at least 95 wt.%, or at least 100 wt.% of ethanol solvate of sitagliptin hydrochloride.

In an embodiment, the deprotection of compound of formula (II) is carried out using a solution of hydrogen chloride in ethanol and the solid product obtained is a crystalline solid comprising substantially sitagliptin hydrochloride ethanol solvate.

In an embodiment, the deprotection of compound of formula (II) is carried out using a solution of hydrochloric acid in ethanol and the solid product obtained is a crystalline solid comprising sitagliptin hydrochloride ethanol solvate and sitagliptin hydrochloride monohydrate.

Optionally, the solid product may be washed with ethanol comprising up to 5% (v/v) of water, wherein the temperature of said solvent is comprised between - 20°C and 50°C, preferably between -5°C and 5°C.

The presence of both compounds can be characterized by their X-ray diffraction pattern. The X-ray diffraction pattern of sitagliptin hydrochloride ethanol solvate (Figure 1) substantially matches the X-ray diffraction pattern of sitagliptin hydrochloride Form IV, disclosed in Figure 5 of WO-A-2011/123641, which was obtained by storing anhydrous sitagliptin hydrochloride in a glass vial under ethanol vapours for 7 days at room temperature. The X-ray diffraction pattern of crystalline sitagliptin hydrochloride monohydrate Form III (Figure 3), corresponds to the crystalline form disclosed in Figure 1 of WO-A-2005/072530.

### Drying step

The crystalline product obtained in step a) of the process of the invention, can be dried in a vacuum drier or using air at atmospheric pressure. In a preferred embodiment, the drying step is carried out in a vacuum drier.

Temperature and vacuum values may be set up by the skilled person in the art within suitable ranges. For example, the drying temperature may be comprised between 50°C and 90°C, preferably between 60°C and 85°C, and more preferably between 65°C and 80°C. The drying vacuum may be comprised between 1 mbar and 100 mbar (corresponding to between 100 Pa and 10 kPa).

The resulting product is crystalline anhydrous sitagliptin hydrochloride, characterized by X-ray diffraction pattern (See Figure 2).

In an embodiment, the anhydrous sitagliptin hydrochloride of formula (III) is used for preparing the sitagliptin hydrochloride monohydrate of formula (IV).

Optionally, a recrystallization step may be performed after the preparation of the anhydrous compound.

### Hydrating step

Crystalline anhydrous sitagliptin hydrochloride obtained in step b) of the process of the invention can be converted into sitagliptin hydrochloride monohydrate by hydration of the former.

The hydrating step may be performed under high relative humidity, or under humid conditions using, for example, an inert gas moistened with water, or water vapours, or wet air.

In an embodiment, the high relative humidity may be comprised between 55% and 100%, preferably between 60% and 95%, and more preferably between 65% and 90%. The hydrating time may be set up by the skilled person in the art according to routine experiments. For example, for a period of time of at least 24 h, preferably at least 36 h, preferably at least 48 h, and more preferably at least 60 h.

A high relative humidity (RH) environment can be obtained in a closed space above saturated solutions of salts or solutions of inorganic acids at specific temperatures, as shown in Table I:

**TABLE I**

| RH (%) | Compound | Temperature (°C) |
|---|---|---|
| 58 | NaBr . 2 H₂O | 20 |
| 65 | Mg(AcO)₂. 4 H₂O | 20 |
| 76 | NaAcO . 3 H₂O | 20 |
| 80 | (MH₄)₂SO₄ | 20 |
| 90 | ZnSO₄ . 7 H₂O | 20 |
| 100 | H₂SO₄ 1 g/cm³ | 20 |

In a preferred embodiment, the hydrating step is performed under humid conditions using an inert gas moistened with water, or water vapours, or wet air.

In a preferred embodiment, the drying step is carried out in a vacuum drier and the hydrating step is carried out under high relative humidity, or under humid conditions using an inert gas moistened with water, or water vapours, or wet air.

In a preferred embodiment, the drying step is carried out in an air drier, i.e., a drier without vacuum, and the hydrating step is carried out under humid conditions using wet air at atmospheric pressure.

When performing the hydrating step under the preferred embodiment of humid conditions using, for example, an inert gas moistened with water, or water vapours, or wet air, the hydrating time may be set up by the skilled person in the art according to routine experiments. For example, for a period of time of at least 24 h, preferably at least 36 h, preferably at least 48 h, and more preferably at least 60 h. Depending on the humid conditions, the hydrating time may be up to 100 h.

In an embodiment the hydrating step is performed in an inert atmosphere with elimination of air, using an inert gas moistened with water. The inert gas may be, for example, argon, helium, methane, carbon dioxide, sulphur hexafluoride; preferably it is argon or carbon dioxide, and more preferably it is argon. In an embodiment, when the drying takes place in a vacuum drier, the depressurization of the vacuum dryer is carried out using gas moistened with water by passing said gas through a water scrubber. Generally, the depressurization step is carried out in a period of time between 1 h and 100 h. In an embodiment, the depressurization may be carried out quickly, i.e., in a period of time below 1 h.

In an embodiment the hydrating step is performed using water vapours. In an embodiment, when the drying takes place in a vacuum drier, the depressurization of the vacuum drier is carried out at a temperature comprised between 0° C and 80° C, preferably between 0° C and 30° C. Generally, the depressurization step is carried out in a period of time comprised between 2 h and 72 h. The pressure in the vacuum drier is maintained between 1 mbar and 200 mbar, preferably between 3 mbar and 50 mbar, and more preferably between 5 mbar and 20 mbar (corresponding to between 100 Pa and 20 kPa, preferably between 300 Pa and 5 kPa, and more preferably between 500 Pa and 2 kPa).

In an embodiment the hydrating step is carried out with wet air. In an embodiment, when the drying takes place in a vacuum drier, the hydrating step is carried out with slow depressurization of the vacuum drier. The relative humidity of the wet air may be comprised between 1% and 100%, preferably between 3% and 50% and more preferably between 5% and 20%. Time of depressurization is selected according to the relative humidity of the wet air. Said time may be comprised between 1 h and 100 h. In an embodiment the period of time is comprised between 1h and 10 h for a relative humidity below 20%. In an embodiment the period of time is comprised between 10 and 30 h, for a relative humidity comprised between 20% and 50%. In an embodiment the period of time is comprised between 30 h and 100 h for a relative humidity higher than 50%.

In an embodiment, when the drying takes place in an air drier using air at atmospheric pressure, the hydrating step is carried out simultaneously with drying. The relative humidity of the air at atmospheric pressure may be comprised between 1% and 100%, preferably between 3% and 50% and more preferably between 5% and 20%. Generally, the time of hydration step is selected according to the used temperature of the air drier. Hydration step should be carried out for at least 4h. The temperature of the drier should be comprised between 30°C and 75°C. In an embodiment the hydration is carried out for at least 8h, for a temperature comprised between 30°C and 60°C. In an embodiment the hydration is carried out for at least 4h, for a temperature comprised between 60°C and 75°C.

Monitoring the formation of crystalline sitagliptin hydrochloride monohydrate of formula (IV) from anhydrous sitagliptin hydrochloride of formula (III) may be carried out by determining the water content of the product, for example, by the Karl Fischer method.

Optionally, a recrystallization step may be performed after the preparation of crystalline sitagliptin hydrochloride monohydrate.

Optionally, the solid product may be washed with ethanol comprising up to 5% (v/v) of water, wherein the temperature of said solvent is comprised between - 20°C and 50°C, preferably between -5°C and 5°C.

Crystalline sitagliptin hydrochloride monohydrate obtained according to the process of the present invention is characterized by its X-ray diffraction pattern (Figure 3). It corresponds to crystalline sitagliptin hydrochloride monohydrate Form III, as disclosed in Figure 1 of WO-A-2005/072530.

### Conditioning step

After hydration and preparation of sitagliptin hydrochloride monohydrate, the product can be further conditioned in containers. Such conditioning requires leaving material after finished process in containers without external interference in its state, to allow product for equalization of its energy state and crystal structure.

The conditioning time may be set up by the skilled person in the art according to routine experiments. For example, for a period of time of at least 24 h, at least 36 h, or at least 48 h.

### Compound of formula (II)

Compound of formula (II), Boc-sitagliptin, (*R*)-*tert*-Butyl (4-oxo-4-(3-(trifluoromethyl)-5,6-dihydro-[1,2,4]triazolo[4,3-a]pyrazin-7(8*H*)-yl)-1-(2,4,5-trifluorophenyl)butan-2-yl)carbamate, CAS Nr. 486460-23-5, is the starting compound in the process of the invention.

It can be prepared according to processes already disclosed in prior art, such as, for example, in WO-A-03/004498.

In an embodiment, compound of formula (II) is prepared according to a process, which comprises:
a) coupling compound of formula (V) 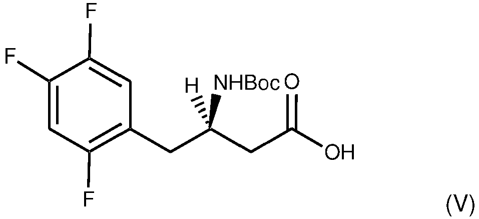
   with compound of formula (VI) 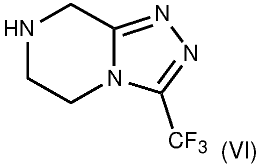
   or an acid addition salt thereof,
   in the presence of an organic base, and ethyl cyano(hydroxyimino)acetate, *N*-(3-dimethylaminopropyl)-*N'*-ethylcarbodiimide or an acid addition salt thereof, as coupling agents, and
b) isolating compound of formula (II) obtained in step a).

In Scheme II is represented the process for preparing compound of formula (II) from compounds of formulae (V) and (VI):

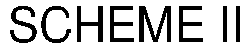

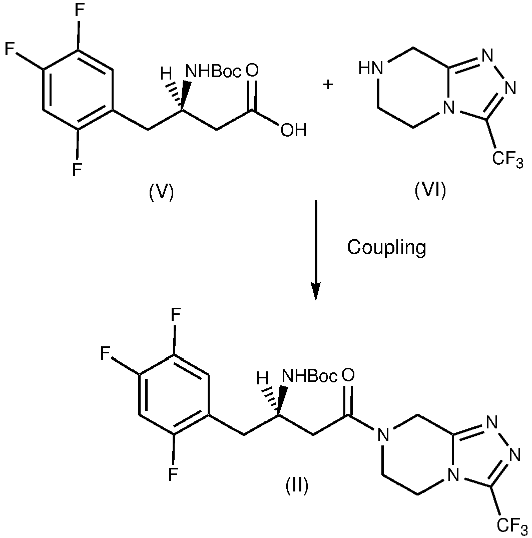

Compound of formula (V) can be prepared as disclosed in prior art, such as, for example, in WO-A-03/004498 (Intermediate 3).

Compound of formula (VI) is commercially available, known in the literature or may be conveniently prepared by a variety of methods familiar to those skilled in the art, such as disclosed in WO-A-03/004498 (Schemes 2 - 5).

Ethyl cyano(hydroxyimino)acetate, CAS Nr. 3849-21-6, is commercially available as, for example, Oxyma Pure Novabiochem^{®} (Sigma-Aldrich), is a non-explosive alternative to hydroxybenzotriazole (HOBt), which can be used in place of HOBt in carbodiimide-mediated coupling reactions.

*N*-(3-dimethylaminopropyl)-*N'*-ethylcarbodiimide hydrochloride, also known as EDC, EDAC, or EDC hydrochloride, CAS Nr. 25952-53-8, is commercially available from, for example, Sigma-Aldrich. It is a water-soluble condensing agent.

In an embodiment, compound of formula (V) and compound of formula (VI) are mixed in isopropanol in the presence of ethyl cyano(hydroxyimino)acetate. Subsequently, a solution of an organic base in isopropanol, and *N*-(3-dimethylaminopropyl)-*N'*-ethylcarbodiimide hydrochloride are added and results a suspension. The organic base can be selected from, for example, triethylamine, diisopropylethylamine, tripropylamine, and triisopropylamine. In a preferred embodiment, the organic base is triethylamine.

The work-up steps can be easily set up by the skilled person in the art by means of routine experiments.

In an embodiment, the isolation of the compound of formula (II) from the reaction mixture comprises heating and/or cooling steps, addition of an anti-solvent, for example, water, and a filtration step.

In an embodiment of step i) of the isolation process, the reaction mixture, which is a suspension, can be stirred for a period of time comprised between 6 h and 24 h, preferably between 6 h and 12 h, and more preferably between 8 h and 10 h, at a temperature may be comprised between 0°C and 40°C, preferably between 10°C and 30°C, and more preferably between 20°C and 25°C.

In an embodiment of step ii) of the isolation process, water is subsequently added to the reaction mixture, which can be heated to a temperature comprised between 50°C and 90°C, preferably between 60°C and 80°C, and more preferably between 70°C and 75°C.

In order to precipitate the product, additional water can be added dropwise to the reaction mixture, as recited in step iii) of the isolation process.

In an embodiment of step iv) of the isolation process, to obtain an improved yield of compound of formula (II) as crystalline solid product, the reaction mixture can be maintained at a temperature between -5°C and 40°C, preferably between -2°C and 20°C, more preferably between -1°C and 10°C, and yet more preferably about 0°C, for a period of time comprised between 6 h and 24 h, preferably between 5 h and 15 h, and more preferably between 5 h and 8 h, and yet more preferably about 4 h.

In an embodiment, the process further may comprise washing the filtered crystalline compound of formula (II) with a mixture of an alcohol and water. In an embodiment it may be used a mixture of isopropanol and water. In an embodiment, it may be used a mixture of isopropanol and water in a ratio of 1:1 (v/v) mixture. In an embodiment, the process includes a further washing with water.

In an embodiment, the process further comprises drying the washed crystalline compound of formula (II).

The drying temperature may be set up to a value comprised between 40°C and 80°C, preferably between 45°C and 65°C, and more preferably about 50°C.

In an embodiment, the drying of the crystalline compound of formula (II) is carried out in an air drier.

In an embodiment, the drying of the crystalline compound of formula (II), can be carried out in a vacuum drier, for example, at a temperature comprised between 60°C and 80°C and a vacuum about 50 mbar (corresponding to 5 kPa).

In an embodiment, the crystalline compound of formula (II) has a XRPD as depicted in Figure 4.

In an embodiment, the crystalline compound of formula (II) is used for preparing the sitagliptin hydrochloride monohydrate of formula (IV).

### Sitagliptin and salts thereof

Sitagliptin of formula (I) or salt thereof, prepared according to the above process, may be included in pharmaceutical compositions, comprising one or more pharmaceutically acceptable excipients or in combination with other active pharmaceutical ingredients and one or more pharmaceutically acceptable excipients.

The invention comprises the following embodiments:
1.- A process for preparing sitagliptin hydrochloride monohydrate of formula (IV) comprising the following steps:
   a) deprotecting compound of formula (II) 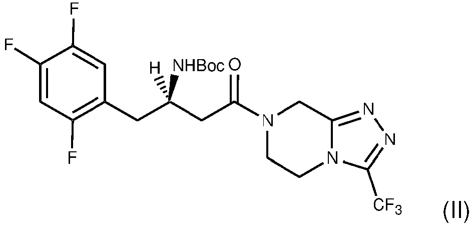 with a solution of hydrochloric acid or hydrogen chloride in a solvent to isolate a crystalline solid product;
   b) drying the crystalline solid product obtained in step a) to obtain crystalline anhydrous sitagliptin hydrochloride of formula (III): 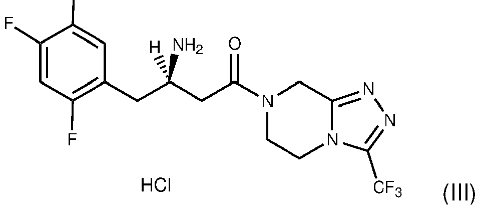
   c) hydrating the product obtained in step b) to obtain crystalline sitagliptin hydrochloride monohydrate of formula (IV) 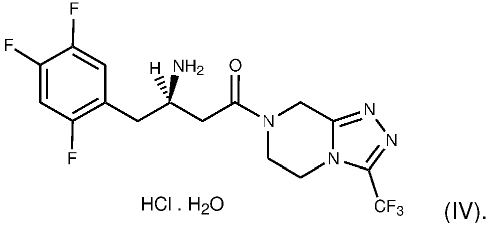
3.- The process according to any one of embodiments 1 or 2, wherein the solvent used in the deprotection step is an organic solvent.
4.- The process according to any one of embodiments 1 to 3, wherein the organic solvent is selected from methanol, ethanol, isopropanol, dichloromethane, ethyl acetate, tetrahydrofuran, dioxane, thiophenol, or mixtures thereof.
5.- The process according to embodiment 4, wherein the organic solvent is ethanol.
6.- The process according to embodiment 5, wherein the deprotection of compound of formula (II) is carried out by dissolving compound of formula (II) in ethanol and adding concentrated hydrochloric acid.
7.- The process according to embodiment 5, wherein the deprotection of compound of formula (II) is carried out by dissolving compound of formula (II) in ethanol and adding a solution of hydrogen chloride in ethanol.
8.- The process according to any one of embodiments 6 or 7, wherein the hydrochloric acid concentration or the hydrogen chloride concentration is comprised between 5% and 37%, preferably between 10% and 35%, more preferably between 31% and 35%, and yet more preferably about 33%.
9.- The process according to any one of embodiments 1 to 8, wherein the isolation of the solid product resulting from the deprotection step comprises seeding the reaction mixture with crystals of sitagliptin hydrochloride monohydrate.
10.- The process according to any one of embodiments 1 to 9, wherein the isolation of the solid product resulting from the deprotection step comprises a combination of at least one heating step, a seeding, at least one cooling step, and filtering of the solid.
11.- The process according to any one of embodiments 3 to 10, wherein the solid product obtained by deprotection compound of formula (II) is a crystalline solid comprising an organic solvate of sitagliptin hydrochloride and optionally sitagliptin hydrochloride monohydrate.
12.- The process according to embodiment 11, wherein the solid product is a crystalline solid comprising sitagliptin hydrochloride ethanol solvate and optionally sitagliptin hydrochloride monohydrate.
13.- The process according to embodiment 12, wherein the crystalline solid usually comprises at least 5 wt.%, at least 10 wt.%, at least 15 wt.%, at least 20 wt.%, at least 25 wt.%, at least 30 wt.%, at least 35 wt.%, at least 40 wt.%, at least 45 wt%., at least 50 wt.%, at least 55 wt.%, at least 60 wt.%, at least 65 wt.%, at least 70 wt.%, at least 75 wt.%, at least 80 wt.%, at least 85 wt.%, at least 90 wt.%, at least 95 wt.%, or at least 100 wt.% of ethanol solvate of sitagliptin hydrochloride.
14.- The process according to any one of embodiments 1 to 13, wherein the crystalline product obtained in step a) is dried in a vacuum drier.
15.- The process according to any one of embodiments 1 to 14, wherein step c) is carried out at a relative humidity comprised between 55% and 100%, preferably between 60% and 95%, and more preferably between 65% and 90%.
16.- The process according to any one of embodiments 1 to 14, wherein step c) is carried out at humid conditions using an inert gas moistened with water, or water vapours, or wet air.
17.- The process according to embodiment 16, wherein the hydrating step is performed in an inert atmosphere with elimination of air, using an inert gas moistened with water.
18.- The process according to embodiment 17, wherein the inert gas is selected from argon, helium, methane, carbon dioxide, and sulphur hexafluoride; preferably it is argon or carbon dioxide, and more preferably it is argon.
19.- The process according to embodiment 16, wherein the hydrating step is performed using water vapours.
20.- The process according to embodiment 19, wherein the drying step is carried out in a vacuum drier, and wherein the hydrating step is performed using water vapours at a temperature comprised between 0° C and 80° C, preferably between 0° C and 30° C, and the depressurization step of the vacuum drier is carried out in a period of time comprised between 2 h and 72 h.
21.- The process according to embodiment 16, wherein the hydrating step is carried out with wet air.
22.- The process according to embodiment 21, wherein the drying step is carried out in a vacuum drier, and wherein the hydrating step is carried out with slow depressurization of the vacuum drier.
23.- The process according to embodiment 21, wherein the drying step is carried out in an air drier at atmospheric pressure, and wherein the hydrating step is simultaneously carried out with wet air at a temperature of the drier between 30°C and 75°C, preferably between 30°C and 60°C.
24.- The process according to any one of embodiments 1 to 23, wherein compound of formula (II) is prepared according to a process, which comprises:
   a) coupling compound of formula (V) 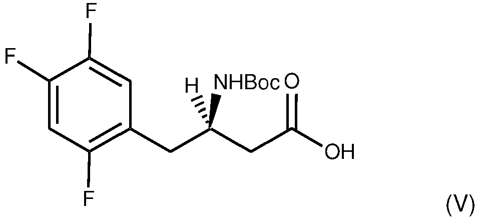
      with compound of formula (VI) 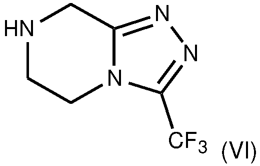
      or an acid addition salt thereof,
      in the presence of an organic base, and ethyl cyano(hydroxyimino)acetate, *N*-(3-dimethylaminopropyl)-*N'*-ethylcarbodiimide or an acid addition salt thereof, as coupling agents, and
   b) isolating compound of formula (II) obtained in step a).
25.- The process according to embodiment 24, wherein the organic base is selected from triethylamine, diisopropylethylamine, tripropylamine, and triisopropylamine.
26.- The process according to embodiment 25, wherein the organic base is triethylamine.
27.- The process according to any one of embodiments 24 to 26, wherein the isolation of the compound of formula (II) from the reaction mixture comprises heating and/or cooling steps, addition of an anti-solvent, and a filtration step.
28.- The process according to any one of embodiments 24 to 27, wherein the crystalline compound of formula (II) is carried out in an air drier or in a vacuum drier.

In the following examples, different embodiments of the invention are provided for illustrative purposes that are understood to be non-limiting.

### Examples

The X-Ray diffractograms were recorded on a Rigaku MiniFlex 600 X-Ray Diffractometer (Bragg-Brentano geometry) with Cu-Kα radiation (1.5406 Å) within the angle range of 2 to 40° 2θ with 0.01° step and scan speed of 6.0 deg/min. Limiting slit was adjusted to 10mm and Kβ filter was used at all times.

### Example 1: Preparation of compound of formula (II)

Into a reactor 10 g (30 mmol) of compound of formula (V), 7.20 g (31.5 mmol) of compound of formula (VI) hydrochloride, 0.98 g (6.9 mmol) of OXYMA and 50 ml of isopropanol were charged. After few minutes of maintenance mixture of 6.07 g (60 mmol) triethylamine in 10 ml of isopropanol was charged dropwise followed by loading of EDC*HCl (8.05 g - 42 mmol). Obtained suspension was stirred for 8 h at 20°C - 25°C. After loading of 20 ml of water reaction mixture was heated to 70°C - 75°C and 60 ml of water was charged dropwise in order to precipitate the product. Reaction mixture was cooled to 0°C during 8 h and stirred at 0°C for 4 h. Obtained product was filtered, washed with isopropanol/water [1:1] mixture (2 x 20 ml), water (20 ml) and dried at 50°C in air drier.

14.0 g (28 mmol, 92%) of compound (II) was obtained.

Figure 4 shows the X-ray diffraction pattern corresponding to crystalline compound of formula (II) obtained according to the above process.

### Example 2: Preparation of sitagliptin hydrochloride monohydrate

Into reactor 10 g (20 mmol) of compound of formula (II), and 140 ml of ethanol were charged. After dissolution HCl solution (2.72 g of 33% HCl (25 mmol) was loaded, reaction mixture was heated to 70°C - 75°C, maintained for 6 h and cooled down to 50°C - 55°C, at which temperature seeding crystals were added. Crystalline suspension was stirred at 50°C - 55°C, for 8 h, and then cooled to 0°C - 5°C at the rate of 5°C - 7°C per hour. After cooling, pH was corrected to the value in range of 2.5 - 2.6 and, after additional 30 minutes of stirring, it was corrected again to value in range of 2.5 - 4.0. Suspension was stirred for next 4 h at -5°C and then resulting precipitate was filtered out and washed two times with 20 ml of EtOH/MTBE (1:1) mixture. It was obtained a mixture of ethanol solvate/monohydrate form of sitagliptin hydrochloride, which was then dried in vacuum drier at 80°C - 90°C/50 mbar, resulting in a crystalline anhydrous form of sitagliptin hydrochloride. This material was transferred to high relative humidity environment (60% to 100%) and kept there for at least 48 h, during which hydration process allowed for conversion to monohydrate form of sitagliptin hydrochloride. After successful hydration, product was transferred to containers in which it was conditioned for at least 48 h.

8.19g (18 mmol, 90%) of crystalline sitagliptin hydrochloride monohydrate, compound of formula (IV) was obtained.

X-ray analysis showed that sitagliptin hydrochloride monohydrate, obtained according to the process of the invention, was Form III, as disclosed in WO-A-2005/072530.

### Example 3: Preparation of sitagliptin hydrochloride monohydrate

Title compound was prepared following substantially the procedure disclosed in Example 2 for deprotection and drying in a vacuum drier.

The dried product was hydrated using wet argon.

After successful hydration, product was transferred to containers in which it was conditioned for at least 48 h.

Crystalline sitagliptin hydrochloride monohydrate, compound of formula (IV) was obtained, showing an impurity content (nitrosamines) of 0.005 - 0.007 ppm, determined using liquid chromatography combined with electrospray ionization tandem mass spectrometry.

X-ray analysis showed that sitagliptin hydrochloride monohydrate, obtained according to the process of the invention, was Form III, as disclosed in WO-A-2005/072530.

### Example 4: Preparation of sitagliptin hydrochloride monohydrate

Title compound was prepared following substantially the procedure disclosed in Example 2 for deprotection and drying in a vacuum drier.

Before depressurization, vacuum drier was connected with water containing vessel, allowing for applying vacuum present in the drier to pull in water vapour from the vessel into the drier space.

The dried product was then hydrated using water vapours.

After successful hydration, product was transferred to containers in which it was conditioned for at least 48 h.

Crystalline sitagliptin hydrochloride monohydrate, compound of formula (IV) was obtained, showing an impurity content (nitrosamines) of about 0.009 - 0.020 ppm, determined using liquid chromatography combined with electrospray ionization tandem mass spectrometry.

X-ray analysis showed that sitagliptin hydrochloride monohydrate, obtained according to the process of the invention, was Form III, as disclosed in WO-A-2005/072530.

### Example 5: Preparation of sitagliptin hydrochloride monohydrate

Title compound was prepared following substantially the procedure disclosed in Example 2 for deprotection and drying in a vacuum drier.

The dried product was hydrated using a slow depressurization with air at atmospheric pressure.

After successful hydration, product was transferred to containers in which it was conditioned for at least 48 h.

Crystalline sitagliptin hydrochloride monohydrate, compound of formula (IV) was obtained, showing an impurity content (nitrosamines) of about 0.019 - 0.027 ppm, determined using liquid chromatography combined with electrospray ionization tandem mass spectrometry.

X-ray analysis showed that sitagliptin hydrochloride monohydrate, obtained according to the process of the invention, was Form III, as disclosed in WO-A-2005/072530.

### Example 6: Preparation of sitagliptin hydrochloride monohydrate

Title compound was prepared following substantially the procedure disclosed in Example 2 for deprotection.

After successful drying and simultaneous hydration with air at atmospheric pressure and relative humidity comprised between 5% and 20%, product was transferred to containers in which it was conditioned for at least 48 h.

Crystalline sitagliptin hydrochloride monohydrate, compound of formula (IV) was obtained, showing an impurity content (nitrosamines) of about 0.013 - 0.019 ppm, determined using liquid chromatography combined with electrospray ionization tandem mass spectrometry.

X-ray analysis showed that sitagliptin hydrochloride monohydrate, obtained according to the process of the invention, was Form III, as disclosed in WO-A-2005/072530.

## Claims

1. A process for preparing sitagliptin hydrochloride monohydrate of formula (IV) comprising the following steps:
a) deprotecting compound of formula (II) 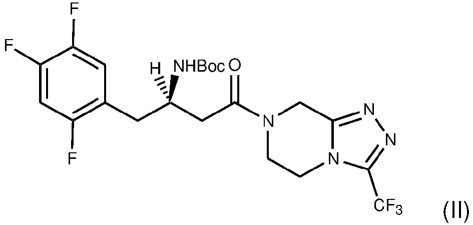 with a solution of hydrochloric acid or hydrogen chloride in a solvent to isolate a crystalline solid product;
b) drying the crystalline solid product obtained in step a) to obtain crystalline anhydrous sitagliptin hydrochloride of formula (III): 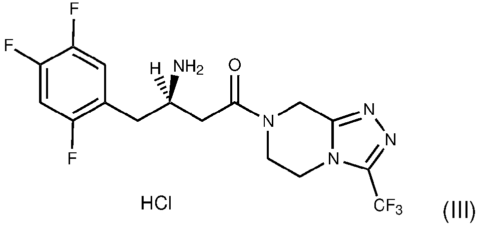
c) hydrating the product obtained in step b) to obtain crystalline sitagliptin hydrochloride monohydrate of formula (IV)

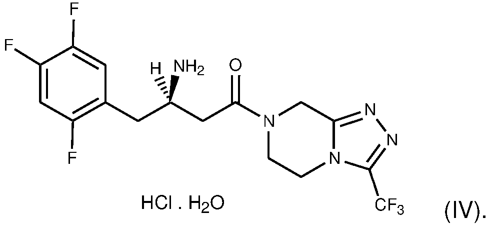

2. The process according to claim 1, wherein the solvent used in the deprotection step is an organic solvent.

3. The process according to claim 1 or 2, wherein the organic solvent is selected from methanol, ethanol, isopropanol, dichloromethane, ethyl acetate, tetrahydrofuran, dioxane, thiophenol, or mixtures thereof.

4. The process according to claim 3, wherein the organic solvent is ethanol.

5. The process according to claim 4, wherein the deprotection of compound of formula (II) is carried out by dissolving compound of formula (II) in ethanol and adding concentrated hydrochloric acid.

6. The process according to claim 4, wherein the deprotection of compound of formula (II) is carried out by dissolving compound of formula (II) in ethanol and adding a solution of hydrogen chloride in ethanol.

7. The process according to any one of claims 1 to 6, wherein the isolation of the solid product resulting from the deprotection step comprises seeding the reaction mixture with crystals of sitagliptin hydrochloride monohydrate.

8. The process according to any one of claims 1 to 7, wherein the isolation of the solid product resulting from the deprotection step comprises a combination of at least one heating step, a seeding, at least one cooling step, and filtering of the solid.

9. The process according to any one of claims 2 to 8, wherein the solid product obtained by deprotection compound of formula (II) is a crystalline solid comprising an organic solvate of sitagliptin hydrochloride and optionally sitagliptin hydrochloride monohydrate.

10. The process according to claim 9, wherein the solid product is a crystalline solid comprising sitagliptin hydrochloride ethanol solvate and optionally sitagliptin hydrochloride monohydrate.

11. The process according to any one of claims 1 to 10, wherein step c) is carried out under high relative humidity, preferably at a relative humidity comprised between 55% and 100%, preferably between 60% and 95%, and more preferably between 65% and 90%, or under humid conditions using an inert gas moistened with water, or water vapours, or wet air.

12. The process according to any one of claims 1 to 11, wherein compound of formula (II) is prepared according to a process, which comprises:
a) coupling compound of formula (V) 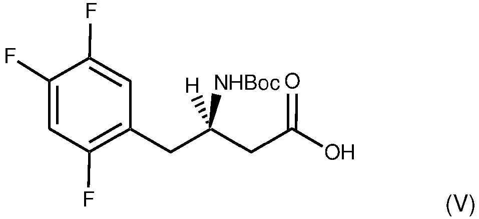
with compound of formula (VI) 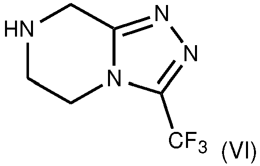
or an acid addition salt thereof,
in the presence of an organic base, and ethyl cyano(hydroxyimino)acetate, *N*-(3-dimethylaminopropyl)-*N'*-ethylcarbodiimide or an acid addition salt thereof, as coupling agents, and
b) isolating compound of formula (II) obtained in step a).

13. The process according to claim 12, wherein the organic base is selected from triethylamine, diisopropylethylamine, tripropylamine, and triisopropylamine.

14. The process according to claim 12 or 13, wherein the isolation of the compound of formula (II) from the reaction mixture comprises heating and/or cooling steps, addition of an anti-solvent, and a filtration step.

## Patentansprüche

1. - Verfahren zur Herstellung von Sitagliptinhydrochlorid-Monohydrat der Formel (IV), umfassend die folgenden Schritte:
a) Entschützen der Verbindung der Formel (II) 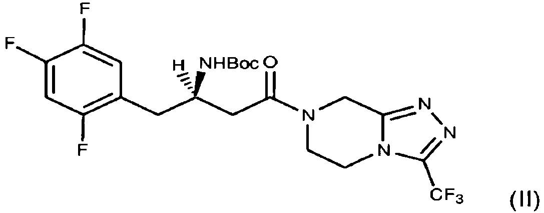 mit einer Lösung von Salzsäure oder Chlorwasserstoff in einem Lösungsmittel, um ein kristallines festes Produkt zu isolieren;
b) Trocknen des in Schritt a) erhaltenen kristallinen Feststoffprodukts, um kristallines wasserfreies Sitagliptinhydrochlorid der Formel (III) zu erhalten: 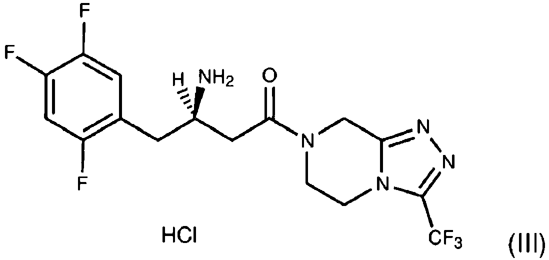
c) Hydratisieren des in Schritt b) erhaltenen Produkts, um kristallines Sitagliptinhydrochlorid-Monohydrat der Formel (IV) zu erhalten

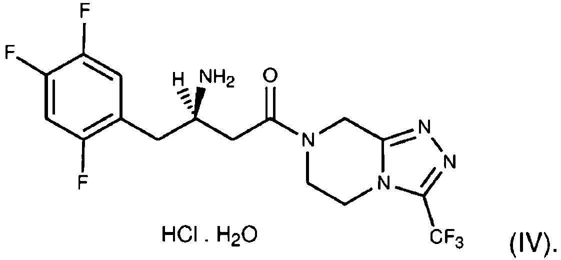

2. - Verfahren nach Anspruch 1, wobei das in Schritt 15 verwendete Lösungsmittel ein organisches Lösungsmittel ist.

3. - Verfahren nach Anspruch 1 oder 2, wobei das organische Lösungsmittel aus Methanol, Ethanol, Isopropanol, Dichlormethan, Ethylacetat, Tetrahydrofuran, Dioxan, Thiophenol oder Mischungen davon ausgewählt ist.

4. - Verfahren nach Anspruch 3, wobei das organische Lösungsmittel Ethanol ist.

5. - Verfahren nach Anspruch 4, wobei die Entschützung der Verbindung der Formel (II) durch Auflösen der Verbindung der Formel (II) in Ethanol und Zugabe von konzentrierter Salzsäure durchgeführt wird.

6. - Verfahren nach Anspruch 4, wobei die Entschützung der Verbindung der Formel (II) durch Auflösen der Verbindung der Formel (II) in Ethanol und Zugabe einer Lösung von Chlorwasserstoff in Ethanol durchgeführt wird.

7. - Verfahren nach einem der Ansprüche 1 bis 6, wobei die Isolierung des aus dem Entschützungs-Schritt resultierenden festen Produkts das Beimpfen des Reaktionsgemisches mit Kristallen von Sitagliptinhydrochlorid-Monohydrat umfasst.

8. - Verfahren nach einem der Ansprüche 1 bis 7, wobei die Isolierung des aus dem Entschützungs-Schritt resultierenden festen Produkts eine Kombination aus mindestens einem ErhitzungsSchritt, einer Impfung, mindestens einem Kühlungs-Schritt und einer Filtrierung des Feststoffs umfasst.

9. - Verfahren nach einem der Ansprüche 2 bis 8, wobei das durch Entschützung der Verbindung der Formel (II) erhaltene feste Produkt ein kristalliner Feststoff ist, der ein organisches Solvat von Sitagliptinhydrochlorid und gegebenenfalls Sitagliptinhydrochlorid-Monohydrat umfasst.

10. - Verfahren nach Anspruch 9, wobei das feste Produkt ein kristalliner Feststoff ist, der ein Ethanol-Solvat von Sitagliptinhydrochlorid und gegebenenfalls Sitagliptinhydrochlorid-Monohydrat umfasst.

11. - Verfahren nach einem der Ansprüche 1 bis 10, wobei Schritt c) unter hoher relativer Luftfeuchtigkeit, vorzugsweise bei einer relativen Luftfeuchtigkeit zwischen 55 % und 100 %, vorzugsweise zwischen 60 % und 95 % und noch bevorzugter zwischen 65 % und 90 %, oder unter feuchten Bedingungen unter Verwendung eines mit Wasser befeuchteten Inertgases, von Wasserdampf oder feuchter Luft durchgeführt wird.

12. - Verfahren nach einem der Ansprüche 1 bis 11, wobei die Verbindung der Formel (II) nach einem Verfahren hergestellt wird, das umfasst:
a) Kupplung der Verbindung der Formel (V) 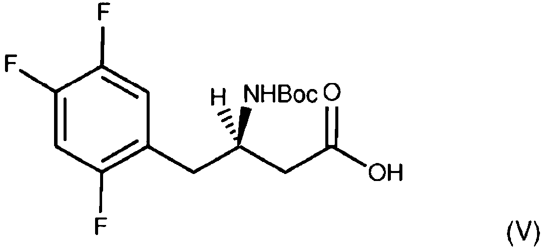
mit einer Verbindung der Formel (VI) 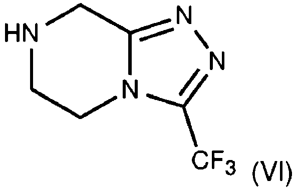
oder eines Säureadditionssalzes davon
in Gegenwart einer organischen Base und Ethyl cyano(hydroxyimino)acetat, N-(3-Dimethylaminopropyl)-N-ethylcarbodiimid oder eines Säureadditionssalzes davon als Kupplungsmittel und
b) Isolieren der in Schritt a) erhaltenen Verbindung der Formel (II).

13. - Verfahren nach Anspruch 12, wobei die organische Base aus Triethylamin, Diisopropylethylamin, Tripropylamin und Triisopropylamin ausgewählt ist.

14. - Verfahren nach Anspruch 12 oder 13, wobei die Isolierung der Verbindung der Formel (II) aus dem Reaktionsgemisch Erhitzungs- und/oder Kühlschritte, die Zugabe eines Antisolvens und einen Filtrationsschritt umfasst.

## Revendications

1. - Procédé de préparation du chlorhydrate de sitagliptine monohydraté de formule (IV) comprenant les étapes suivantes :
a) déprotection du composé de formule (II) 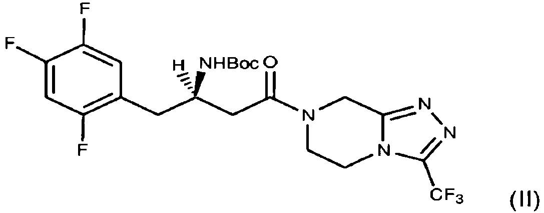 avec une solution d'acide chlorhydrique ou de chlorure d'hydrogène dans un solvant pour isoler un produit solide cristallin ;
b) séchage du produit solide cristallin obtenu à l'étape a) pour obtenir du chlorhydrate de sitagliptine anhydre cristallin de formule (III): 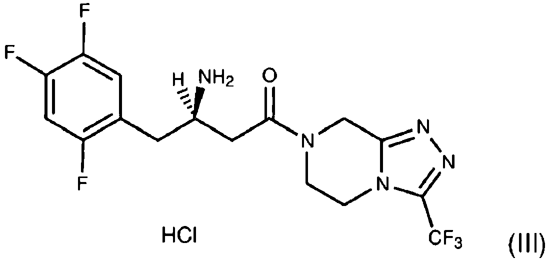
c) hydratation du produit obtenu à l'étape b) pour obtenir du chlorhydrate de sitagliptine monohydraté cristallin de formule (IV)

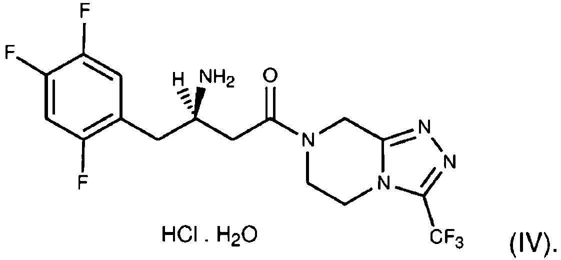

2. - Procédé selon la revendication 1, dans lequel le solvant utilisé dans l'étape de déprotection 15 est un solvant organique.

3. - Procédé selon la revendication 1 ou 2, dans lequel le solvant organique est choisi parmi le méthanol, l'éthanol, l'isopropanol, le dichlorométhane, l'acétate d'éthyle, le tétrahydrofurane, le dioxane, le thiophénol ou leurs mélanges.

4. - Procédé selon la revendication 3, dans lequel le solvant organique est l'éthanol.

5. - Procédé selon la revendication 4, dans lequel la déprotection du composé de formule (II) est effectuée en dissolvant le composé de formule (II) dans de l'éthanol et en ajoutant de l'acide chlorhydrique concentré.

6. - Procédé selon la revendication 4, dans lequel la déprotection du composé de formule (II) est effectuée en dissolvant le composé de formule (II) dans de l'éthanol et en ajoutant une solution d'acide chlorhydrique dans de l'éthanol.

7. - Procédé selon l'une quelconque des revendications 1 à 6, dans lequel l'isolement du produit solide résultant de l'étape de déprotection comprend l'ensemencement du mélange réactionnel avec des cristaux de chlorhydrate de sitagliptine monohydraté.

8. - Procédé selon l'une quelconque des revendications 1 à 7, dans lequel l'isolement du produit solide résultant de l'étape de déprotection comprend une combinaison d'au moins une étape de chauffage, un ensemencement, au moins une étape de refroidissement et une filtration du solide.

9. - Procédé selon l'une quelconque des revendications 2 à 8, dans lequel le produit solide obtenu par déprotection du composé de formule (II) est un solide cristallin comprenant un solvate organique de chlorhydrate de sitagliptine et éventuellement du chlorhydrate de sitagliptine monohydraté.

10. - Procédé selon la revendication 9, dans lequel le produit solide est un solide cristallin comprenant un solvate d'éthanol de chlorhydrate de sitagliptine et, éventuellement, du chlorhydrate de sitagliptine monohydraté.

11. - Procédé selon l'une quelconque des revendications 1 à 10, dans lequel l'étape c) est réalisée sous une humidité relative élevée, de préférence à une humidité relative comprise entre 55 % et 100 %, de préférence entre 60 % et 95 %, et plus préférablement entre 65 % et 90 %, ou dans des conditions humides en utilisant un gaz inerte humidifié avec de l'eau, ou de la vapeur d'eau, ou de l'air humide.

12. - Procédé selon l'une quelconque des revendications 1 à 11, dans lequel le composé de formule (II) est préparé selon un procédé qui comprend :
a) le couplage du composé de formule (V) 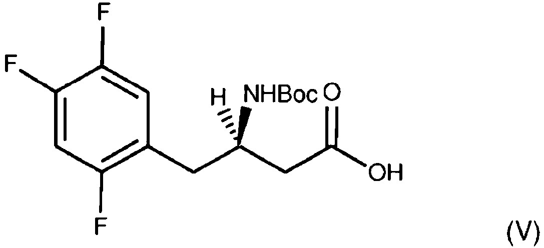
avec le composé de formule (VI) 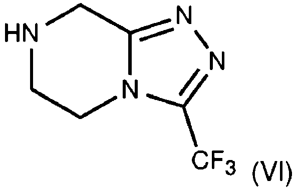
ou un sel d'addition d'acide de celui-ci,
en présence d'une base organique, et l'éthyl cyano(hydroxyimino)acétate, N-(3-diméthylaminopropyl)-N-éthylcarbodiimide ou un sel d'addition d'acide de celui-ci, comme agents de couplage, et
b) isoler le composé de formule (II) obtenu à l'étape a).

13. - Procédé selon la revendication 12, dans lequel la base organique est choisie parmi la triéthylamine, la diisopropylethylamine, la tripropylamine et la triisopropylamine.

14. - Procédé selon la revendication 12 ou 13, dans lequel l'isolement du composé de formule (II) à partir du mélange réactionnel comprend des étapes de chauffage et/ou de refroidissement, l'ajout d'un anti-solvant et une étape de filtration.
